# EUROPEAN PATENT SPECIFICATION

(11) **EP 0 581 826 B1**
(45) Date of publication and mention of the grant of the patent: **20.09.1995**
(21) Application number: 92909120.5
(22) Date of filing: 22.04.1992
(51) Int. Cl.: C11D 17/00, A61L 9/01

(54) **LAVATORY CLEANSING BLOCKS**
TABLETTEN ZUM REINIGEN VON TOILETTEN
BLOCS DE NETTOYAGE DE WC

(30) Priority: 22.04.1991 GB 9108574
(43) Date of publication of application: 09.02.1994
(73) Proprietor: JEYES LIMITED, Norfolk IP24 1HA (GB)
(72) Inventor: MARSHALL, John, Norwich Norfolk NR4 6AE (GB); BLACK, Steven, John, Farrell, Attleborough Norfolk NR17 2HS (GB); WILSON, Brian, Murie, Bressingham Diss Norfolk IP22 2BB (GB); YOUNGS, James, William, Thetford Norfolk IP24 1JG (GB)
(74) Representative: Lamb, John Baxter
(86) International application number: GB9200741
(87) International publication number: WO9218605

(56) References cited:
- EP-A- 0 184 416
- EP-A- 0 206 725
- EP-A- 0 341 836
- EP-A- 0 462 643
- GB-A- 2 021 143

## Description

This invention is concerned with improvements in and relating to lavatory cleansing blocks.

In particular, the present invention is concerned with solid lavatory cleansing blocks intended to be brought into contact with the flush water of a lavatory or urinal whereby a part of the block is dissolved in the flush water to release active ingredients thereto for cleaning the lavatory or urinal. The blocks are immersed in the water cistern of a lavatory or urinal, either as a free-standing block or may be placed or contained in a dispensing device, to be sited in a lavatory cistern. The invention is also concerned with lavatory cleansing blocks for intermittent contact with the flush water of a lavatory or urinal, e.g., a so-called "rim block" for placing in a container to be held under the rim of a lavatory.

One common class of component of lavatory cleansing blocks comprises one or more water-soluble surface active agents. Another desirable component of such blocks would be a halogen release agent, that is a compound which on contact with water releases hypohalous acid and/or hypohalite ions to the water, since these are powerful sanitising and cleansing agents. In principle, there would appear to be no problem in combining these two classes of ingredient in a single block. However, halogen release agents are, by their nature, powerful chemically reactive species, serving as halogenating or oxidising agents. Thus, in practice, we have found that halogen release agents (i) tend to react with surface active materials and/or (ii) tend, when moistened, to evolve gas thereby losing their activity and, in many cases, destroying the physical integrity of the cleansing block. Further, halogen release agents may attack component parts of lavatories, urinals or their cisterns.

A particularly useful class of chlorine release agents comprises the N-chlorinated cyanuric acid derivatives such as sodium dichloroisocyanurate and trichlorisocyanuric acid. We have found in practice, however, that it is generally just not practically possible to reproducibly and reliably incorporate such chlorine release agents in a lavatory cleansing block in amounts sufficient to give useful cleansing and/or sanitising, e.g. amounts of 10% by weight or more.

We have now found, in accordance with the present invention, that it is possible to include such chlorinated cyanuric acid derivatives in blocks which contain no added filler (water-soluble or otherwise), other than those which may be introduced as contaminants or components of a surface active component.

According to the invention, therefore, there is provided a solid lavatory cleansing block formed of a composition comprising (A) from 5 to 80, preferably from 10 to 70%, by weight of a surface active component comprising one or more anionic surface active agents; (B) from 10 to 75, preferably from 15 to 60% by weight of a chlorine release agent component consisting of one or more chlorinated cyanuric acid derivative chlorine release agents; and (C) from 2 to 25%, preferably from 3 to 20%, by weight of a solubility control agent (as hereinafter defined); the composition containing not more than 20% by weight of water-soluble inorganic salts introduced with the anionic surface active agent(s) and containing no added fillers or diluents.

Suitable anionic surface active agents for use in the blocks of the invention include alkali metal, typical sodium, paraffin sulphonates; alkali metal alkyl sulphates and alkali metal alkyl aryl sulphonates, especially alkali metal benzene sulphonates. A typical example is sodium dodecyl benzene sulphonate which is a readily available material of commerce. The anionic surface active component of the block forms from 5 to 80% by weight of the composition, preferably from 10 to 70 % by weight thereof, most preferably from 25 to 65 % by weight thereof, and especially from 40 to 60% by weight thereof.

The chlorine release component of the block is an N-chlorinated cyanuric acid derivative, such as sodium dichloroisocyanurate or trichloroisocyanuric acid, especially the former.

The chlorine release component is present in the blocks of the invention in an amount of from 10 to 75% by weight, preferably from 15 to 70 % by weight, more preferably from 20 to 50 % by weight, and especially from 30 to 40% by weight.

The third component of the block is a solubility control agent, that is, a compound of lower solubility than the anionic surface active component and which assists in controlling the rate of dissolution of the block.

The solubility control agent may be virtually wholly insoluble in water or if, as discussed below a nonionic surface active agent, have a low HLB, e.g. 5 or less. Such agents should be resistant to attack by the chlorine release component, both in the composition and in aqueous solutions produced by dissolution of the composition in use. It is a matter of simple experiment to determine whether any candidate is so resistant. Generally, the solubility control agent should be a saturated organic material or a highly chlorinated organic material. Examples of solubility agents which may be employed include polyethylene waxes; fatty alcohols; fatty acids; low ethoxylates (e.g. containing up to 4 ethylene oxide units per mole) of fatty alcohols and alkylphenols; paradichlorobenzene; and difficultly hydrolysable esters such as methyl salicylate and isobornyl acetate.

The solubility control agent should form from 2 to 25% of the weight of the block, more preferably from 3 to 20%, more preferably from 5 to 15% and especially 6 to 12% thereof.

Certain of the solublility control agents noted above, the ethoxylated fatty alcohols and alkyl phenols, also possess surface active properties and thus may contribute to the overall cleansing effect of a composition containing them. In this connection it may be noted that other nonionic surfactants may be present.

The blocks of the invention must not contain more than 20% by weight of inert water-soluble salts, such as sodium sulphate, present as impurities introduced with the anionic surface active agent which is preferably not more than 75% and especially less than 10% by weight thereof. Commercially available anionic surface active agents often contain appreciable amounts of filler or diluent, such as sodium sulphate or sodium phosphate, and such commercially available materials may be used in formulating blocks in accordance with the invention to provided that in so doing too much salt is not introduced. However, in accordance with the invention, substantially no additional (e.g. less than 1% by weight) water-soluble or other filler should be introduced, e.g., sodium sulphate, sodium carbonate, sodium tripolyphosphate, sodium bicarbonate, sodium metasilicate, sodium sesquicarbonate, sodium chloride, clays, calcite or the like.

As will be appreciated, any other ingredient present in the composition of the invention should be resistant to attack by the chlorine release agent. Thus, for example, most dyestuffs commonly employed in lavatory cleansing blocks to impart a pleasant colouration to the flush water are not sufficiently resistant to the chlorine release agents with the results that (a) the dyestuffs are decolourised or discoloured to an unpleasant colour and (b) available halogen, which would otherwise serve as a sanitizing agent, is lost. However, there are indications that Acid Blue 7 may be adequately resistant. Similarly, most perfumes which are commonly employed in lavatory cleansing blocks are also subject to attack by the chlorine release agents although some odiferous materials may be adequately resistant (and additionally serve as solubility control agents); examples of these being substituted quinolines, cedryl methyl ether and cineole.

Lavatory cleansing blocks commonly contain a germicide or preservative but this is not generally necessary in the case of the blocks of the invention since they already contain powerful germicides, namely the halogen release agents.

As noted above, it is not generally possible to incorporate dyestuffs or perfumes in the blocks of the invention. However, some insoluble pigments are resistant to the chlorine release agents and may be incorporated in the blocks of the invention to impart a colouration to the flush water. Examples of suitable pigments include copper phthalocyanine pigments which can be conveniently incorporated in the blocks of the invention in the forms of dispersions in suitable media.

The blocks of the invention are suitably formed by a compression process, especially an extrusion process comprising the steps of forming a mixture of the components of the composition, extruding this mixture into rod or bar form and then cutting the extruded rod or bar into appropriately sized pieces or blocks. (In this connection it may be noted that a free standing lavatory cleansing block suitably has a weight of from 20 to 150 gms, preferably from 30 to 100 gms).

When an extrusion process is employed the mixture to be extruded should contain up to 15% by weight, of a liquid component or a solid component which is liquefied under extrusion conditions to act as a processing aid. In the case of the blocks of the invention this is conveniently provided by the use of a liquid solubility control agent such as a lower ethoxylated alcohol or alkyl phenol; a higher alcohol, chlorinated hydrocarbon or mineral oil.

The invention also provides a method of cleansing a lavatory by intacting the flush water thereof with a block in accordance with the invention.

In order that the invention may be well understood the following examples are given by way of illustration only.

### Examples

Blocks having the formulations shown in Table 1 were produced by extruding the mixture and cutting into blocks, having a weight of about 65 gm.

Each block was tested in a lavatory cistern by subjecting it, to a total of 17 flushes per day. The life of each block was calculated by counting the total number of flushes which it survived. As will be seen, the blocks in accordance with the invention (Examples 1, 2 and 3) had significantly greater life than those not in accordance with the invention (Examples 4-11).

**Table 1**

| (Content of blocks in weight percent) | | | | | | | | | | | |
|---|---|---|---|---|---|---|---|---|---|---|---|
| Example | 1 | 2 | 3 | 4 | 5 | 6 | 7 | 8 | 9 | 10 | 11 |
| Marlon A390 | | 62.0 | 41.5 | 40.5 | 40.5 | 40.5 | 40.5 | 40.5 | 40.5 | 40.5 | 25.7 |
| Nansa HS80GPF | | | 20.0 | 20.0 | 20.0 | 20.0 | 20.0 | 20.0 | 20.0 | 20.0 | 33.3 |
| Nansa HS85S | 61.0 | - | - | - | - | - | - | - | - | - | - |
| Ficlor | 30.0 | 30.0 | 30.0 | 30.0 | 30.0 | 30.0 | 30.0 | 30.0 | 30.0 | 30.0 | 30.0 |
| Dobanol 91 | - | 7.0 | 7.5 | 7.5 | 7.5 | 7.5 | 7.5 | 7.5 | 7.5 | 7.5 | - |
| Synperonic AZ | 9.0 | - | - | - | - | - | - | - | - | - | - |
| Mineral Oil | - | 1.0 | 1.0 | 1.0 | 1.0 | 1.0 | 1.0 | 1.0 | 1.0 | 1.0 | |
| Arkopal 040 | - | - | - | - | - | - | - | - | - | - | 9.0 |
| Sodium Sulphate | - | - | - | 1.0 | - | - | - | - | - | - | - |
| Sodium Tripolyphosphate | - | - | - | - | 1.0 | - | - | - | - | - | - |
| Sodium metasilicate | - | - | - | - | - | 1.0 | - | - | - | - | - |
| Sodium Carbonate | - | - | - | - | - | - | 1.0 | - | - | - | - |
| Sodiumsesqulcarbonate | - | - | - | - | - | - | - | 1.0 | - | - | - |
| Sodium bicarbonate | - | - | - | - | - | - | - | - | 1.0 | - | - |
| Sodium Chloride | - | - | - | - | - | - | - | - | - | 1.0 | - |
| Smectite Clay | - | - | - | - | - | - | - | - | - | - | 2.0 |
| Life Testing/ Number of Flushes | >400 | >400 | >400 | 220 | 145 | 61 | 38 | 48 | 133 | 132 | 85 |

### Chemical Names

- Marlon A 390: is a trade name of Huls Aktiengesellschaft. Contains approx 90% dodecylbenzene sulphate sodium salt, the balance being mainly sodium sulphate
- Nansa HS80SGPF: is a trade mark of Albright and Wilson Ltd. Contains approx 80% dodecylbenzene sulphonate sodium salt, the balance being mainly magnesium sulphate and sodium sulphate.
- Ficlor: is a trade name of Fisons plc Common chemical name sodium dichloroisocyanurate dihydrate.
- Dobanol 91: is a trade name of Shell Chemicals. It is a distillation of C₉ to C₁₁ primary alcohols contains approximately C₉ alcohol (17.25%), C₁₀ alcohol (38.75%) and C₁₁ alcohol (29.5%) the balance being other alkanols.
- Synperonic AZ: is a trade mark of ICI Ltd. It is an approx 99% active ethoxylated synthetic primary alcohol with 2 molecules of ethylene oxide.
- Arkopal N040: is a trade name of Hoescht UK Ltd. It is a approx 99% active alkylphonol ethoxylate with 4 moles of ethylene oxide.

## Claims

1. A solid lavatory cleansing block formed of a Composition comprising (A) from 5% to 80% by weight of a surface active component Comprising one or more anionic surface active agents; (B) from 10 to 75% by weight of a chlorine release agent component consisting of one or more chlorinated cyanuric acid derivative chlorine release agents; and (C) from 2 to 25% by weight of a solubility control agent (as hereinbefore defined); the composition Containing not more than 20% by weight of water-soluble inorganic salt introduced with the anionic surface active agent component and containing no other added fillers or diluents,

2. A Composition as claimed in claim 1 comprising from 10 to 20% by weight of anionic surface component; from 15 to 60% by weight of chlorine release component and from 3 to 20% by weight of solubility control agent.

## Patentansprüche

1. Fester Toilettenreinigungsblock, der aus einer Zusammensetzung geformt wird, die folgende Elemente aufweist: (A) zwischen 5 und 80 Gew.-% einer oberflächenaktiven Komponente, die ein oder mehrere anionische oberflächenaktive Mittel umfaßt; (B) zwischen 10 und 75 Gew.-% einer Komponente aus einem chlorfreisetzenden Mittel, die aus einem oder mehreren chlorierten Cyanursäurederivat-Chlorfreisetzungsmitteln besteht; und (C) zwischen 2 und 25 Gew.-% eines Mittels zur Steuerung der Löslichkeit (wie es vorstehend definiert wurde); wobei die Zusammensetzung nicht mehr als 20 Gew.-% an wasserlöslichem anorganischen Salz enthält, das mit der Komponente des anionischen oberflächenaktiven Mittels eingeführt wird, und keine anderen zugesetzten Füllstoffe oder Verdünnungsmittel enthält.

2. Zusammensetzung nach Anspruch 1, die zwischen 10 und 20 Gew.-% der anionischen Oberflächenkomponente, zwischen 15 und 60 Gew.-% der chlorfreisetzenden Komponente und zwischen 3 und 20 Gew.-% des die Löslichkeit steuernden Mittels enthält.

## Revendications

1. Bloc solide de nettoyage de WC formé d'une composition comprenant (A) de 5% à 80% en poids d'un composant tensio-actif comprenant un ou plusieurs agents tensio-actifs anioniques; (B) de 10 à 75% en poids d'un composant d'un agent de libération de chlore, composé de un ou plusieurs agents de libération de chlore dérivés de l'acide cyanurique chloré; et (C) de 2 à 25% en poids d'un agent de commande de la solubilité (comme défini ci-dessus); la composition ne contenant pas plus de 20% en poids de sel inorganique soluble dans l'eau introduit avec ledit composant d'agent tensio-actif anionique et ne contenant pas d'autres agents de remplissage ou de diluants ajoutés.

2. Composition selon la revendication 1, comprenant de 10 à 20% en poids d'un composant de surface anionique, de 15 à 60% en poids d'un composant de libération de chlore et de 3 à 20% en poids d'un agent de commande de la solubilité.
